# EUROPEAN PATENT APPLICATION

(11) **EP 4 537 754 A1**
(43) Date of publication of application: **16.04.2025**
(21) Application number: 23820096.8
(22) Date of filing: 07.06.2023
(51) Int. Cl.: A61B 5/256, A61B 5/28, A61B 5/291, A61B 5/00, A61B 5/266

(54) **WEARABLE DEVICE CAPABLE OF MEASURING EEG AND ECG FOR LONG TIME**

(30) Priority: 07.06.2022 KR 20220069046; 16.11.2022 KR 20220153739
(71) Applicant: SK Biopharmaceuticals Co., Ltd., Seongnam-si, Gyeonggi-do 13494 (KR)
(72) Inventor: PARK, Chan Ho, Seongnam-si, Gyeonggi-do 13494 (KR); CHAE, Woongsin, Seongnam-si, Gyeonggi-do 13494 (KR); KIM, Yongwook, Seongnam-si, Gyeonggi-do 13494 (KR); CHUNG, Raeeun, Seongnam-si, Gyeonggi-do 13494 (KR)
(74) Representative: Vossius & Partner Patentanwälte Rechtsanwälte mbB
(86) International application number: PCT/KR2023/007786
(87) International publication number: WO 2023/239160

(57) **Abstract**

A wearable device comprises a sensor unit and a control unit. The sensor unit: includes a plurality of electrodes which are mounted on the head of a user above the cervical portion and acquire electrical signals from the head; acquires an electroencephalogram of the user on the basis of the difference between the electronic signals acquired by two of the plurality of electrodes; and acquires an electrocardiogram of the user on the basis of the difference between the electrical signals acquired by two other electrodes among the plurality of electrodes. The control unit is electrically connected with the sensor unit, and converts, into digital signals, the analog signals of the electroencephalogram and electrocardiogram acquired by the sensor unit.

## Description

### [Technical Field]

### CROSS-REFERENCE TO RELATED APPLICATION

This application claims priority to and the benefit of Korean Patent Application No. 10-2022-0069046 filed in the Korean Intellectual Property Office on June 7, 2022 and Korean Patent Application No. 10-2022-0153739 filed in the Korean Intellectual Property Office on November 16, 2022, the entire contents of which are incorporated herein by reference.

The present disclosure relates to a wearable device, and more particularly, to a wearable device capable of collecting biosignals such as electroencephalography (EEG) and electrocardiogram (ECG).

### [Background Art]

Living organisms, including humans, generate various electrical signals that can be collectively called biosignals. The biosignals can include electroencephalography (EEG), electrocardiogram (ECG), ballistocardiogram (BCG), and photoplethysmogram (PPG), and by analyzing these biosignals, various information about the state of the living organism can be obtained.

Among various biosignals, brain waves and electrocardiograms are signals that inform the state of the brain and heart, which are the most important organs for human survival, and are by far the most important biosignals.

In general, EEG is acquired from the user's head, and ECG is acquired from the chest. Therefore, in order to implement a device that measures brain waves and electrocardiograms simultaneously, a head-mounted sensor for measuring biosignals and a chest-mounted sensor must be separately provided. This complicates the configuration of the device, increases its bulk, and makes it inconvenient for users to use the device.

### [Disclosure]

### [Technical Problem]

The present disclosure attempts to provide a wearable device which is capable of simultaneously measuring electroencephalography (EEG) and electrocardiogram (ECG) of a user, and limits a body part to be measured to a narrow area to simplify components of a device for measurement, and miniaturize and light-weight the components, and more conveniently measure both the EEG and ECG.

### [Technical Solution]

A wearable device according to an embodiment includes a sensor unit and a control unit. The sensor unit includes a plurality of electrodes which are mounted on the head of a user above the cervical portion, and detect electrical signals from the head, detects an electroencephalogram of the user based on a difference between electrical signals detected by two of the plurality of electrodes, and detects an electrocardiogram of the user based on a difference between electrical signals detected by two other electrodes among the plurality of electrodes. The control unit is electrically connected with the sensor unit, and converts, into digital signals, analog signals of the electroencephalogram and electrocardiogram detected by the sensor unit.

The sensor unit may include an electroencephalography (EEG) acquisition unit that acquires the electroencephalography (EEG) of the user and an electrocardiogram (ECG) acquisition unit that acquires the electrocardiogram (ECG) of the user. The plurality of electrodes may include an active electrode, a reference electrode, a ground electrode, and two electrocardiogram (ECG) electrodes.

The active electrode may include two frontal lobe electrodes and two temporal lobe electrodes. The electroencephalography (EEG) acquisition unit may acquire electroencephalography (EEG)s based on the difference between the electrical signal detected by the active electrode and the electrical signal detected by the reference electrode.

On the other hand, the active electrode may include a first active electrode having two frontal lobe electrodes, and a second active electrode having two temporal lobe electrodes. When the head of the user is divided into left and right regions, a measurement position of each of the first active electrode and the second active electrode may belong to at least one of the left region and the right region.

The electroencephalography (EEG) acquisition unit may acquire electroencephalography (EEG)s of a left brain or a right brain or both brains for the frontal lobe based on the difference between the electrical signal detected by the first active electrode and the electrical signal detected by the reference electrode, and acquires electroencephalography (EEG) of at least one of the left brain or the right brain or both brains for the temporal lobe based on the difference between the electrical signal detected by the second active electrode and the electrical signal detected by the reference electrode.

Two electrocardiogram electrodes may be located in the left region and the right region of the head, respectively, and the electrocardiogram (ECG) acquisition unit may acquire an electrocardiogram (ECG) based on the difference between the electrical signals detected by two electrocardiogram electrodes, respectively.

Two frontal lobe electrodes may be located at either positions Fp1 and Fp2 or positions F7 and F8. Two temporal lobe electrodes may be located at either positions T3 and T4 or positions T5 and T6. The reference electrode may be located closer to the ground than two temporal lobe electrodes, with a central axis of the head being perpendicular to the ground. The plurality of electrodes may be composed of hydrogel electrodes.

The sensor unit may further include an acceleration sensor, and an acceleration acquisition unit measuring 3-axis position information according to the user's movement using the acceleration sensor. The sensor unit may include a first electrode support, and a second electrode support extended from the first electrode support toward the user's forehead. The plurality of electrodes may be mounted on the first electrode support and the second electrode support, and the first electrode support and the second electrode support may be made of a flexible material.

The control unit may include a filter unit filtering an output signal of the sensor unit, an analog-to-digital conversion unit converting a filtered analog output signal into a digital signal, and a communication unit transmitting the converted digital signal to a mobile device.

The sensor unit may be connected to the control unit through a connection line, and the control unit may be connected to the mobile device through a wired connection unit. The sensor unit and the control unit may receive power from the mobile device. On the other hand, the wearable device may further include a power supply supplying the power to the sensor and the control unit. The communication unit may be a wireless communication unit including at least one of Bluetooth, WiFi, 4th generation mobile communication, and 5th generation mobile communication, and the control unit may be connected to the mobile device through the wireless communication unit.

The sensor unit and the control unit may be utilized for monitoring, diagnosing, and predicting central nervous system diseases.

### [Advantageous Effects]

According to an embodiment of the present disclosure, electroencephalography (EEG) and electrocardiogram (ECG) of a user can be simultaneously measured, and a body part to be measured is limited to a narrow area to simplify components of a device for measurement, and miniaturize and light-weight the components, and both the EEG and ECG can be more conveniently measured.

### [Description of the Drawings]

FIG. 1 is a diagram illustrating a configuration of a wearable device according to a first embodiment.
FIG. 2 is a diagram illustrating the configuration of the wearable device and a wearing state of the user according to the first embodiment.
FIG. 3 is a partial enlarged diagram of FIG. 2.
FIG. 4 is a perspective view illustrating a sensor unit illustrated in FIG. 3.
FIG. 5 is a diagram illustrating installation locations of a plurality of electrodes.
FIG. 6 is a perspective view illustrating a control unit in the wearable device illustrated in FIG. 2.
FIG. 7 is a diagram illustrating a configuration of a wearable device according to a second embodiment.
FIG. 8 is a diagram illustrating a wearable device according to the second embodiment implemented in a neck band form.
FIG. 9 is a perspective of the wearable device illustrated in FIG. 7.
FIG. 10 is a perspective view of the wearable device according to the second embodiment implemented in a form of a hairband.
FIG. 11 is a perspective view of the wearable device according to the second embodiment implemented in a form of glasses.
FIGS. 12 and 13 are perspective views of the wearable device according to the second embodiment implemented in a form of an earphone.

### [Mode for Invention]

The present disclosure will be described more fully hereinafter with reference to the accompanying drawings, in which embodiments of the invention are shown. As those skilled in the art would realize, the described embodiments may be modified in various different ways, all without departing from the scope of the present disclosure.

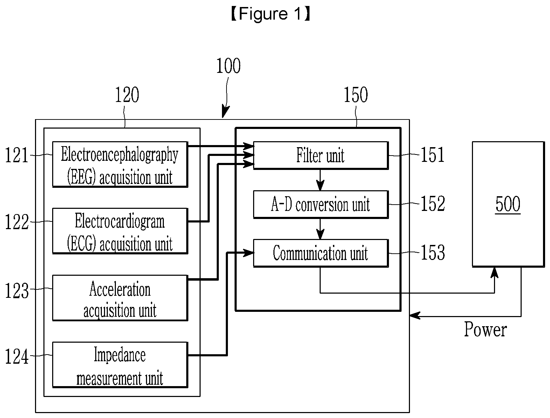

FIG. 1 is a diagram illustrating a configuration of a wearable device according to a first embodiment.

Referring to FIG. 1, the wearable device 100 of the first embodiment is a device that simultaneously measures biosignals including electroencephalography (EEG) and electrocardiogram (ECG) from the user's head, and is optionally capable of measuring acceleration. In the explanation below, 'acceleration' may be all replaced with 'angular velocity'.

The electroencephalography (EEG) is an electrical signal generated by brain activity. The electroencephalography (EEG) may be used to diagnose brain diseases such as epilepsy, stroke, and brain tumors, and recently, the electroencephalography (EEG) are also being used to monitor brain activity of subjects, such as for testing perceptual and cognitive abilities, in addition to diagnosing diseases.

The electrocardiogram (ECG) is an electrical signal generated by the contraction and relaxation of the heart, and serves as data for interpreting the operation of the heart. By observing the electrocardiogram (ECG), the speed and consistency of the heartbeat may be found, and through this, the occurrence of heart-related diseases such as myocardial infarction, angina pectoris, and arrhythmia may be determined.

Acceleration is the three-axis acceleration in the forward, backward, left, right, and up-down directions due to the user's movement, and the degree of the user's movement may provide information about the time of error in biosignal detection. Therefore, the user's movements may be measured using acceleration information.

In general, the electroencephalography (EEG) is acquired from the user's head, the electrocardiogram (ECG) is acquired from the user's chest, and acceleration is acquired from the wrist, but the wearable device 100 according to the first embodiment may measure both the electroencephalography (EEG) and the electrocardiogram (ECG) from the user's head, and optionally measure accelerations together. In the wearable device (100) of the first embodiment, the user's head may be the head above the cervical portion.

The wearable device 100 of the first embodiment includes a sensor unit 120 that measures a biosignal and a control unit 150 electrically connected to the sensor unit 120. The control unit 150 receives the biosignal measured by the sensor unit 120, converts an analog signal into a digital signal, and inputs the digital signal into a mobile device 500.

The sensor unit 120 includes an electroencephalography (EEG) acquisition unit 121 that acquires the electroencephalography (EEG) of the user and an electrocardiogram (ECG) acquisition unit 122 that acquires the electrocardiogram (ECG) of the user. Each of the electroencephalography (EEG) acquisition unit 121 and the electrocardiogram (ECG) acquisition unit 122 may be composed of a plurality of electrodes that detect electrical signals from the head above the user's neck, and the electroencephalography (EEG) and the electrocardiogram (ECG) may be acquired based on a difference between electrical signals detected by two of the plurality of electrodes.

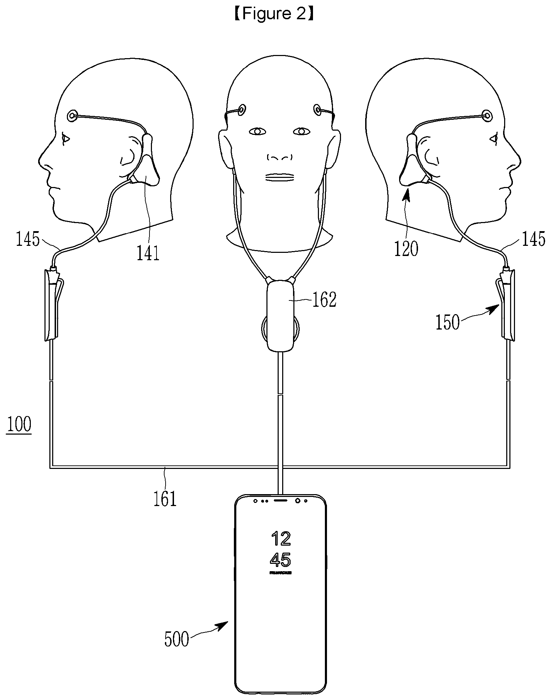

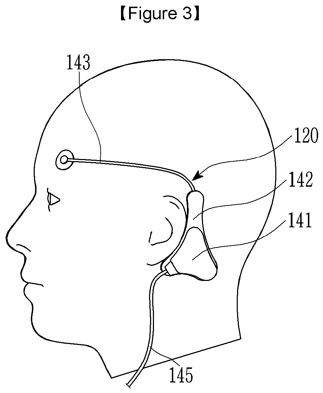

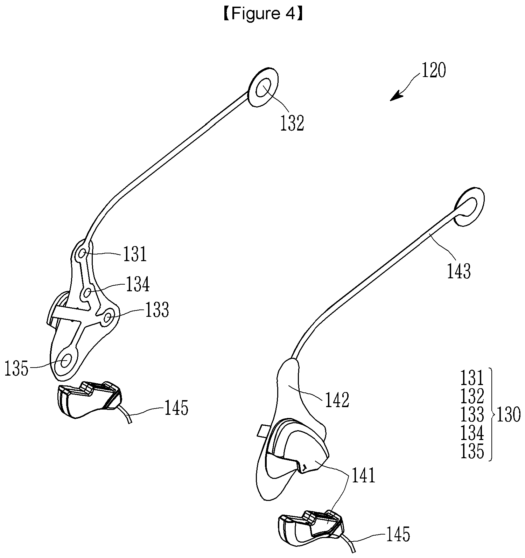

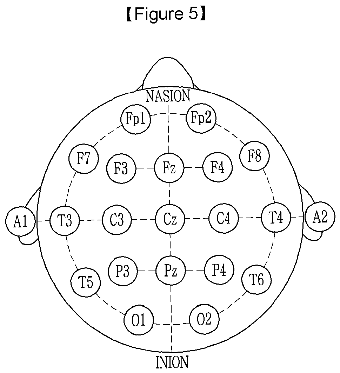

FIG. 2 is a diagram illustrating the configuration of the wearable device and a wearing state of the user according to the first embodiment, and FIG. 3 is a partially enlarged diagram of FIG. 2. FIG. 4 is a perspective view illustrating a sensor unit illustrated in FIG. 3, and FIG. 5 is a diagram illustrating installation locations of a plurality of electrodes. In FIG. 4, a pair of sensor parts have the same configuration.

Referring to FIGS. 1 to 5, the electroencephalography (EEG) acquisition unit 121 may collect biosignals by two temporal lobe electrodes 131 and two frontal lobe electrodes 132. In an embodiment, the electroencephalography (EEG) acquisition unit 121 may include an active electrode, a reference electrode 133, and a ground electrode 134, and two temporal lobe electrodes 131 and two frontal lobe electrodes 132 may be active electrodes.

The active electrodes may detect electrical signals at a first position on a skin surface of the user's forehead and at a second position on the skin surface of the user's temporal portion. The first position may be positions Fp1 and Fp2 of FIG. 5, and the second position may be positions T3 and T4 of FIG. 5. Depending on a skull structure and size and the lesion site of a subject, the first position may be positions F7 and F8 in FIG. 5, and the second position may be positions T5 and T6 in FIG. 5.

The ground electrode 134 may detect an electrical signal at a third position on the skin surface of the temporal portion, and the reference electrode 133 may detect an electrical signal at a fourth position on the skin surface of the temporal portion. The second position and the third position may be present on a skin surface of any one of a mastoid of a user. The fourth position may be located further down from the ground than the second position, with a central axis of the user's head being perpendicular to the ground.

The ground electrode 134 is for detecting electrical signals of other electrodes, and the electrical signal detected by the active electrode described below means a voltage difference between the active electrode and the ground electrode. The electrical signal detected by electrodes other than the active electrode also means a voltage difference between the electrode and the ground electrode 134.

The electroencephalography (EEG) acquisition unit 121 may acquire electroencephalography (EEG)s based on the difference between the electrical signal detected by the active electrode and the electrical signal detected by the reference electrode 133, and may use 2 to 4 channels. At this time, the difference between the two electrical signals may be a raw electroencephalography (EEG), and the electroencephalography (EEG) may be acquired through additional signal processing.

In another embodiment, the electroencephalography (EEG) acquisition unit 121 may include a first active electrode, a second active electrode, and a reference electrode. The two frontal lobe electrodes 132 may be the first active electrodes, and the two temporal lobe electrodes 131 may be the second active electrodes.

The first active electrode may detect an electrical signal at a fifth position on the skin surface of the user's forehead, and the second active electrode may detect an electrical signal at a sixth position on the skin surface of the user's temporal portion. The reference electrode 133 may detect an electrical signal at a seventh position on the skin surface of the user's temporal portion.

The fifth, sixth, and seventh positions may be the same as the first, second, and fourth positions described above, respectively. When dividing the user's forehead and temporal portion into left and right regions, the fifth and sixth positions may belong to at least one of the left and right regions.

In this case, the electroencephalography (EEG) acquisition unit 121 may acquire electroencephalography (EEG)s of a left brain or a right brain or both brains for the frontal lobe based on the difference between the electrical signal detected by the first active electrode and the electrical signal detected by the reference electrode 133, and may acquire electroencephalography (EEG)s of the left brain or the right brain or both brains for the temporal lobe based on the difference between the electrical signal detected by the second active electrode and the electrical signal detected by the reference electrode 133.

In both of the above-described embodiments, the electrocardiogram (ECG) acquisition unit 122 may include two electrocardiogram (ECG) electrodes 135. Two electrocardiogram electrodes 135 may detect the electrical signal on the skin surface of the user's temporal portion. When dividing the user's head into the left and right regions, any one of the two electrocardiogram electrodes 135 is located in the left region and the other one is located in the right region. The electrocardiogram (ECG) electrode 135 may be located at a distance from the reference electrode 133 along a direction parallel to the ground.

The electrocardiogram (ECG) acquisition unit 122 may acquire an electrocardiogram (ECG) based on the difference between the electrical signal detected by any one of the two electrocardiogram (ECG) electrodes 135 and the electrical signal detected by the other electrocardiogram (ECG) electrode. Any one of the two electrocardiogram electrodes 135 may be referred to as an active electrode for electrocardiogram measurement, and the other one may be referred to as a reference electrode for electrocardiogram measurement.

The sensor unit 120 may include a pair of sensor unit bodies 141, a first electrode support 142 coupled to each of the pair of sensor unit bodies 141, a second electrode support 143 extended from the first electrode support 142 toward the user's forehead, and a plurality of electrodes 130 located on an inner side of each of the first and second electrode supports 142 and 143 and in contact with the user's skin surface. The plurality of electrodes 130 are a plurality of electrodes that constitute the above-described electroencephalography (EEG) acquisition unit 121 and electrocardiogram (ECG) acquisition unit 122, and each of the first and second electrode support units 142 and 143 may be made of a flexible material that may be easily bent according to a curvature of the user's skin.

The sensor unit body 141 may be formed in a structure to be separated into two parts. Any one of the two parts may be connected to the first and second electrode supports 142 and 143, and the other one may be connected to a connection line 145 described below.

The plurality of electrodes 130 may be composed of wet electrodes, and specifically, may be composed of hydrogel-type electrodes. The wet electrodes have less friction noise and signal noise than dry electrodes, and may collect signals stably by closely contacting the skin. In an embodiment, each of the plurality of electrodes 130 may include a hydrogel layer in contact with the skin, a conductive material layer located on the inside of the hydrogel layer and functioning as an actual electrode, a connector terminal located on the inside of the conductive material layer, and a non-conductive material layer located on the inside of the connector terminal.

The sensor unit 120 may include an acceleration sensor, and an acceleration acquisition unit 123 that measures 3-axis position information according to the user's movement using the acceleration sensor. The acceleration sensor may be mounted inside the sensor unit body 141.

The acceleration sensor provides a direct current (DC) component proportional to a magnitude of a gravity acceleration acting in a direction perpendicular to a plane on which the acceleration sensor is placed. Further, when the acceleration sensor accelerates in the direction perpendicular to the plane on which the acceleration sensor is placed, the acceleration sensor provides an alternating current (AC) component proportional to a magnitude of a motion acceleration of a component perpendicular to the plane. An output of the DC component of the acceleration sensor may provide information on a tilting degree of the acceleration sensor.

The acceleration acquisition unit 123 may determine a shape of the user's head movement by using a tilting angle of the acceleration sensor acquired from the output of the acceleration sensor. Further, the acceleration acquisition unit 123 may determine the degree of the user's head movement from the output of the alternating current (AC) component of the acceleration sensor.

The sensor unit 120 may further include an impedance measurement unit 124. The impedance measuring unit 124 may measure a contact impedance between each of the plurality of electrodes 130 and the user's skin in contact with each of the plurality of electrodes 130, and output a signal for identifying an electrode among the plurality of electrodes 130, in which the contact impedance exceeds a predetermined value according to a predetermined method.

The user may wear the sensor unit 120 of the above-described configuration at a position adjacent to at least one of both ears, and the plurality of electrodes 130 may be maintained in a skin attachment state by the first and second support units 142 and 143. The plurality of electrodes 130 may be integrally coupled to the sensor unit body 141 by the first and second supports 142 and 143, or may be configured to be easily and repeatedly attached and detached from the sensor unit body 141 by a physical device or a permanent magnet device. For example, the plurality of electrodes 130 may be coupled to wired connector terminals and configured to be easily and repeatedly attached and detached as needed by the user.

The sensor unit 120 and the control unit 150 may be connected by a connection line 145. The electroencephalography (EEG) and the electrocardiogram (ECG) acquired by the sensor unit 120, and analog signals of the acceleration acquired selectively may be delivered to the control unit 150 through the connection line 145, and output as a digital type electroencephalography (EEG) output signal, and an electrocardiogram (ECG) output signal and an acceleration output signal, respectively. The control unit 150 may include a filter unit 151, an analog-to-digital (A-D) conversion unit 152, and a communication unit 153, and may be connected to a mobile device via a connection unit.

The filter unit 151 may perform filtering by applying a band-pass filter having a predetermined first pass band to the electroencephalography (EEG) output signal of the sensor unit 120, and perform filtering by applying a band-pass filter having a predetermined second pass band to the electrocardiogram (ECG) output signal. The control unit 150 may process the biosignal through software to more accurately extract a feature of the biosignal, such as by emphasizing the feature of the biosignal or removing noise other than the biosignal through the filter unit 151.

The analog-to-digital conversion unit 152 applies a first sampling rate and a second sampling rate to the electroencephalography (EEG) output signal and electrocardiogram output signal filtered by the filter unit 151, respectively, and converts the analog signal into the digital signal. At this time, the first sampling rate may be determined based on a maximum frequency of the first pass band, and the second sampling rate may be determined based on a maximum frequency of the second pass band.

The communication unit 153 transmits the electroencephalography (EEG) output signal and the electrocardiogram (ECG) output signal converted into the digital signals to the mobile device 500 through the connection unit at a first transmission speed and a second transmission speed, respectively. A ratio between the first transmission speed and the second transmission speed may be determined based on a ratio between the first sampling rate and the second sampling rate.

The connection unit connecting the control unit 150 and the mobile device 500 may be a wired connection unit 161. The control unit 150 may be integrally connected to the wired connection terminal of the mobile device 500 using the wired connection unit 161, or may be connected to any position of a cable connecting the sensor unit 120 and the wired connection terminal of the mobile device 500. The control unit 150 may transmit the electroencephalography (EEG) output signal, the electrocardiogram (ECG) output signal, and the acceleration output signal converted into the digital signal to the mobile device 500, and transmit the output signals to a server system using a wireless communication module of the mobile device 500.

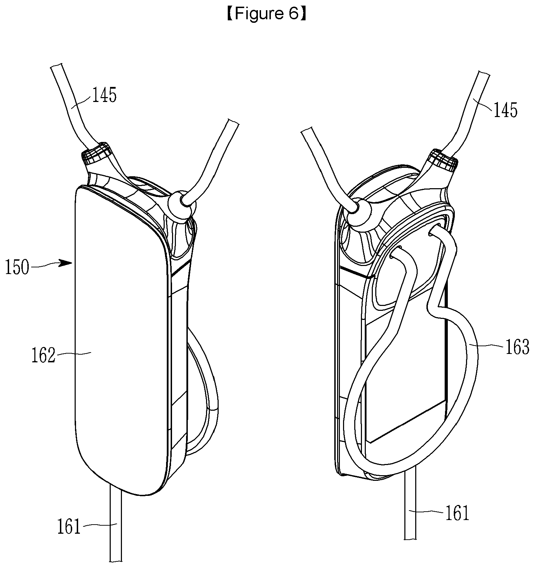

FIG. 6 is a perspective view illustrating a control unit in the wearable device illustrated in FIG. 2.

Referring to FIGS. 1 and 6, the control unit 150 includes a control unit body 162 that accommodates the filter unit 151, the analog-to-digital conversion unit 152, and the communication unit 153. A wearing means such as a clip 163 may be provided to the control unit body 162. The control unit main body 162 may be connected to a pair of sensor unit bodies 141 by a pair of connection lines 145 and connected to the mobile device 500 by the wired connection unit 161.

The wearable device 100 of the first embodiment may not include a power supply required for driving the sensor unit 120 and the control unit 150, and may receive power from the mobile device 500 through the wired connection unit 161. Therefore, the wearable device 100 of the first embodiment is capable of continuously measuring biosignals for a long period of time, such as 12 hours, and the overall configuration may be made miniaturized and lightweight, so it is very easy to use in daily life.

Next, a wearable device according to a second embodiment is described.. Except the wearable device of the second embodiment is a wireless type, the wearable device of the second embodiment is formed in the same or similar configuration as the first embodiment. Below, configurations that are different from the first embodiment will be primarily described.

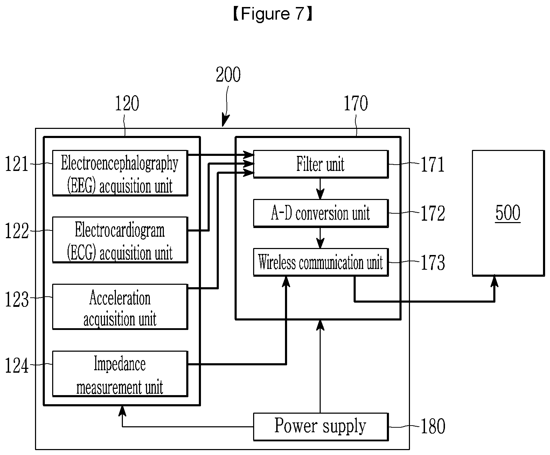

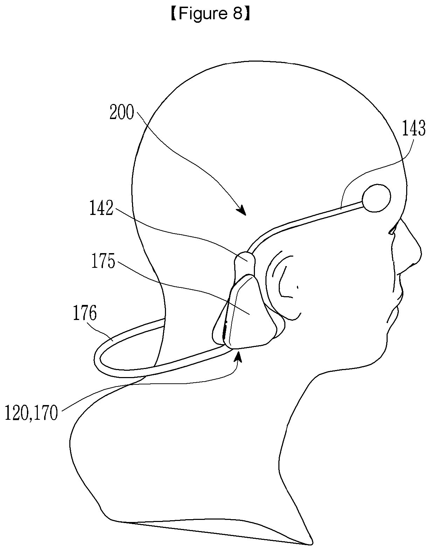

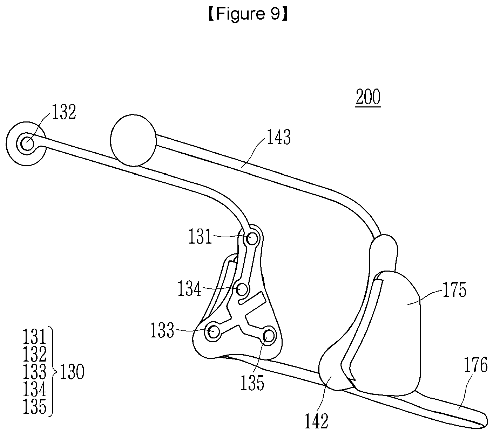

FIG. 7 is a diagram illustrating a configuration of a wearable device according to a second embodiment and FIG. 8 is a diagram illustrating a wearable device according to the second embodiment implemented in a neck band form. FIG. 9 is a perspective of the wearable device illustrated in FIG. 7.

Referring to FIGS. 7 to 9, the wearable device 200 of the second embodiment includes a sensor unit 120 that measures a biosignal and a control unit 170 electrically connected to the sensor unit 120. The control unit 170 receives the biosignal measured by the sensor unit 120, converts an analog signal into a digital signal, and transmits the digital signal into a mobile device 500 using a wireless communication unit 163.

The sensor unit 120 may include an electroencephalography (EEG) acquisition unit 121, an electrocardiogram (ECG) acquisition unit 122, an acceleration acquisition unit 123, and an impedance measurement unit 124. The electroencephalography (EEG) acquisition unit 121 may collect biosignals by two temporal lobe electrodes 131 and two frontal lobe electrodes 132.

In an embodiment, the electroencephalography (EEG) acquisition unit 121 may include an active electrode, a reference electrode 133, and a ground electrode 134, and two temporal lobe electrodes 131 and two frontal lobe electrodes 132 may be active electrodes. In another embodiment, the electroencephalography (EEG) acquisition unit 121 may include a first active electrode, a second active electrode, and the reference electrode 133. The two frontal lobe electrodes 132 may be the first active electrodes, and the two temporal lobe electrodes 131 may be the second active electrodes. In both of the embodiments, the electrocardiogram (ECG) acquisition unit 122 may include two electrocardiogram (ECG) electrodes 135. In the sensor unit 120, a process in which the electroencephalography (EEG) acquisition unit 121 acquires electroencephalography (EEG) and a process in which the electrocardiogram (ECG) acquisition unit 122 acquires electrocardiogram (ECG) are the same as those of the above-described first embodiment.

The sensor unit 120 may include a pair of first electrode supports 142, a second electrode support 143 extended from the first electrode support 142 toward the user's forehead, and a plurality of electrodes 130 located on an inner side of each of the first and second electrode supports 143 and in contact with the user's skin surface. The plurality of electrodes 130 may be composed of wet electrodes, and specifically, may be composed of hydrogel-type electrodes.

The acceleration acquisition unit 123 measures 3-axis position information according to the user's movement using an acceleration sensor. The sensor unit 120 and the control unit 170 may share one main body 175, and the acceleration sensor may be mounted inside the main body 175. The control unit 170 may include a filter unit 171, an analog-to-digital conversion unit 172, and a wireless communication unit 173, and may be connected to a mobile device 500 by a wireless connection unit 173. The configurations and operations of the filter unit 171 and the analog-to-digital (A-D) conversion unit 172 are the same as those in the above-described first embodiment.

The wireless communication unit 173 may include at least one of Bluetooth which is a short-range wireless technology standard, Wireless Fidelity (WiFi) which is a wireless LAN, 4th generation mobile communication (LTE: Long Term Evolution), and 5th generation mobile communication (5G: 5^{th} Generation).

The control unit 170 may transmit an electroencephalography (EEG) output signal, an electrocardiogram (ECG) output signal, and an acceleration output signal converted into the digital signals to a wireless communication module of the mobile device 500 through the wireless communication unit 173, and transmit the output signals to a server system using the wireless communication module of the mobile device 500. Using software installed in the mobile device 500, an electrical signal received from the control unit 170 may be analyzed and transmitted to a server, and recording of biosignals and transmission to the server may occur in real time.

The wearable device 200 of the second embodiment includes a power supply 180 required for driving the sensor unit 120 and the control unit 170. The power supply 180 may be configured as a typical battery, but is not limited to such an example.

In FIG. 8, the wearable device is configured in the form of a neckband, and a pair of main bodies 175 may be integrally connected by a semicircular connecting member 176 that wraps around the back of the user's neck. The wearable device in the form of the neckband is an integrated configuration in which the sensor unit and the control unit share a single main body 175, which is advantageous in miniaturizing the entire wearable device. The wearable device 200 of the second embodiment may be implemented in a form that is easy to use in daily life, such as a hairband, glasses, and earphones, in addition to the neckband form.

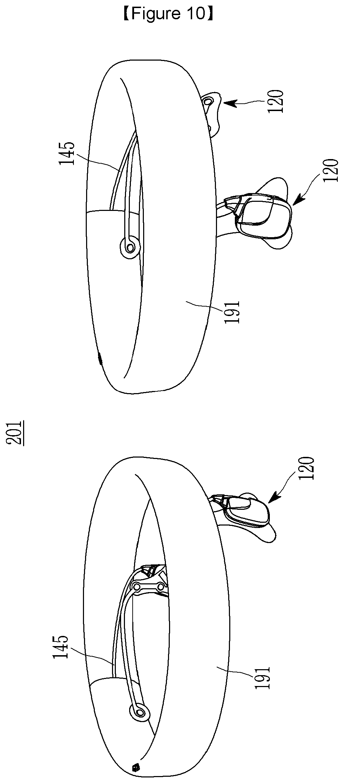

FIG. 10 is a perspective view of the wearable device according to the second embodiment implemented in a hairband form. In FIG. 10, the wearable device 201 includes a hairband unit 191 that surrounds the user's head so as to be in close contact with the user's forehead and back of the head. The sensor unit 120 is fixedly installed in the hairband unit 191 so that a plurality of electrodes come into contact with the user's skin, and the control unit may be installed inside the hairband unit 191. The sensor unit 120 and the control unit may be connected by a connection line 145.

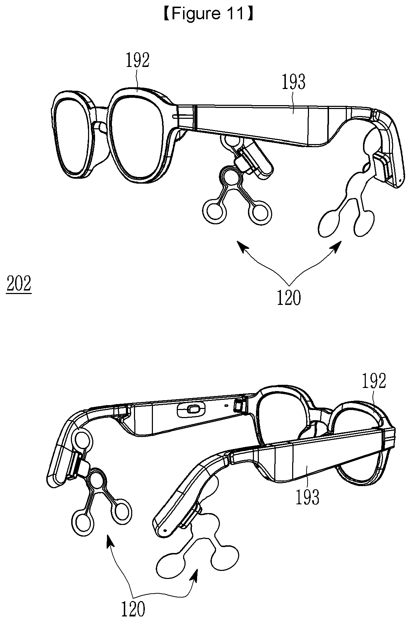

FIG. 11 is a perspective view of the wearable device according to the second embodiment implemented in a form of glasses. In FIG. 11, the wearable device 202 includes a glasses frame 192 and glasses temples 193. The sensor unit 120 is fixedly installed at the end of the glasses temples 193 so that the plurality of electrodes come into contact with the user's skin, and the control unit may be installed inside the glasses temples 193.

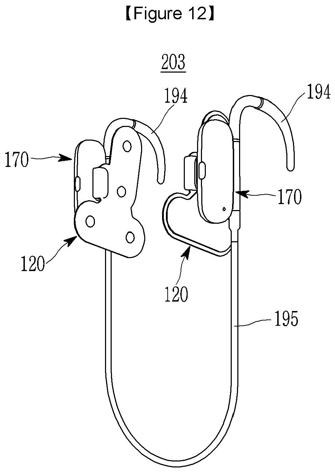

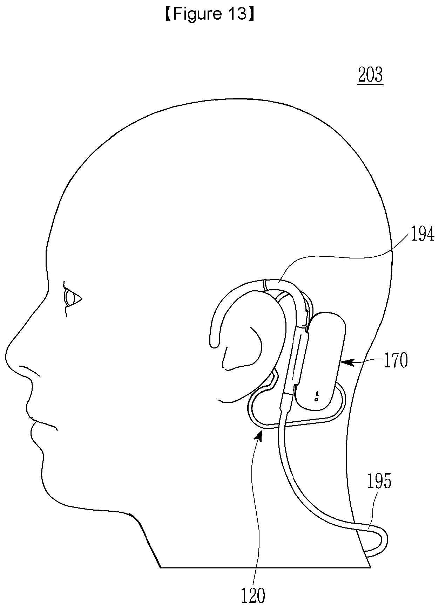

FIGS. 12 and 13 are perspective views of the wearable device according to the second embodiment implemented in a form of an earphone. In FIGS. 12 and 13, the wearable device 203 includes a ring 194 that may be worn on the user's ear. The sensor unit 120 and the control unit 170 are installed and connected to the ring 194, and a pair of rings 194 may be connected integrally by a wire 195. The wearable device 203 in the form of earphones has a structure in which the prefrontal lobe electrodes of the embodiments are omitted, but frontal lobe electroencephalography (EEG) may be measured by a method such as installing a separate patch.

The wearable devices 100, 200, 201, 203, and 203 according to the first and second embodiments described above may be utilized for diagnosing and predicting brain diseases such as epilepsy, stroke, and brain tumors, and can be utilized for monitoring, diagnosing, and predicting symptoms of various central nervous system diseases such as attention deficit hyperactivity disorder (ADHD), autism, depression, sleep disorders, consciousness disorders, stress, and dementia.

Although a preferred embodiment of the present disclosure is described hereinabove, the present disclosure is not limited thereto, and various modifications can be made within the scopes of the claims, and the detailed description of the present disclosure and the accompanying drawings, and belongs to the scope of the present disclosure, of course.

## Claims

1. A wearable device comprising:
a sensor unit including a plurality of electrodes which are mounted on the head of a user above the cervical portion, and detect electrical signals from the head, detecting an electroencephalogram of the user based on a difference between electrical signals detected by two of the plurality of electrodes, and detecting an electrocardiogram of the user based on a difference between electrical signals detected by two other electrodes among the plurality of electrodes; and
a control unit electrically connected with the sensor unit, and converts, into digital signals, analog signals of the electroencephalogram and electrocardiogram detected by the sensor unit.

2. The wearable device of claim 1, wherein:
the sensor unit includes that acquires the electroencephalography (EEG) of the user and an electrocardiogram (ECG) acquisition unit that acquires the electrocardiogram (ECG) of the user, and
the plurality of electrodes include an active electrode, a reference electrode, a ground electrode, and two electrocardiogram (ECG) electrodes.

3. The wearable device of claim 2, wherein:
the active electrode includes two frontal lobe electrodes and two temporal lobe electrodes, and
the electroencephalography (EEG) acquisition unit acquires electroencephalography (EEG)s based on the difference between the electrical signal detected by the active electrode and the electrical signal detected by the reference electrode.

4. The wearable device of claim 2, wherein:
the active electrode includes a first active electrode having two frontal lobe electrodes, and a second active electrode having two temporal lobe electrodes, and
when the head of the user is divided into left and right regions, a measurement position of each of the first active electrode and the second active electrode belongs to at least one of the left area and the right area.

5. The wearable device of claim 4, wherein:
the electroencephalography (EEG) acquisition unit acquires electroencephalography (EEG)s of a left brain or a right brain or both brains for the frontal lobe based on the difference between the electrical signal detected by the first active electrode and the electrical signal detected by the reference electrode, and acquires electroencephalography (EEG)s of the left brain or the right brain or both brains for the temporal lobe based on the difference between the electrical signal detected by the second active electrode and the electrical signal detected by the reference electrode.

6. The wearable device of claim 2, wherein:
two electrocardiogram electrodes are located in the left region and the right region of the head, respectively, and
the electrocardiogram (ECG) acquisition unit acquires an electrocardiogram (ECG) based on the difference between the electrical signals detected by two electrocardiogram electrodes, respectively.

7. The wearable device of claim 3 or 4, wherein:
two frontal lobe electrodes are located at either positions Fp1 and Fp2 or positions F7 and F8,
two temporal lobe electrodes are located at either positions T3 and T4 or positions T5 and T6, and
the reference electrode is located closer to the ground than two temporal lobe electrodes, with a central axis of the head being perpendicular to the ground.

8. The wearable device of claim 2, wherein:
the plurality of electrodes are composed of hydrogel electrodes.

9. The wearable device of claim 2, wherein:
the sensor unit further includes an acceleration sensor, and an acceleration acquisition unit measuring 3-axis position information according to the user's movement using the acceleration sensor.

10. The wearable device of claim 2, wherein:
the sensor unit includes a first electrode support, and a second electrode support extended from the first electrode support toward the user's forehead,
the plurality of electrodes are mounted on the first electrode support and the second electrode support, and
the first electrode support and the second electrode support are made of a flexible material.

11. The wearable device of claim 2, wherein:
the control unit includes a filter unit filtering an output signal of the sensor unit, an analog-to-digital conversion unit converting a filtered analog output signal into a digital signal, and a communication unit transmitting the converted digital signal to a mobile device.

12. The wearable device of claim 11, wherein:
the sensor unit is connected to the control unit through a connection line,
the control unit is connected to the mobile device through a wired connection unit, and
the sensor unit and the control unit receive power from the mobile device.

13. The wearable device of claim 11, further comprising:
a power supply supplying the power to the sensor and the control unit,
wherein the communication unit is a wireless communication unit including at least one of Bluetooth, WiFi, 4th generation mobile communication, and 5th generation mobile communication, and
the control unit is connected to the mobile device through the wireless communication unit.

14. The wearable device of claim 1, wherein:
the sensor unit and the control unit are utilized for monitoring, diagnosing, and predicting central nervous system diseases.
